# EUROPEAN PATENT SPECIFICATION

(11) **EP 4 607 228 B1**
(45) Date of publication and mention of the grant of the patent: **18.11.2026**
(21) Application number: 24158598.3
(22) Date of filing: 20.02.2024
(51) Int. Cl.: G01R 33/56, G01R 33/50, G16H 30/40, A61B 6/00

(54) **METHOD FOR PROCESSING MEASUREMENT INFORMATION TO SELECT A RESPECTIVE LABEL FOR MULTIPLE VOXELS OR PIXELS OF A MEDICAL IMAGE DATASET, DATA PROCESSING SYSTEM, COMPUTER PROGRAM, AND COMPUTER READABLE MEDIUM**
VERFAHREN ZUR VERARBEITUNG VON MESSDATEN ZUR AUSWAHL EINES JEWEILIGEN LABELS FÜR MEHRERE VOXEL ODER PIXEL EINES MEDIZINISCHEN BILDDATENSATZES, DATENVERARBEITUNGSSYSTEM, COMPUTERPROGRAMM UND COMPUTERLESBARES MEDIUM
PROCÉDÉ DE TRAITEMENT D'INFORMATIONS DE MESURE POUR SÉLECTIONNER UNE ÉTIQUETTE RESPECTIVE POUR DE MULTIPLES VOXELS OU PIXELS D'UN ENSEMBLE DE DONNÉES D'IMAGES MÉDICALES, SYSTÈME DE TRAITEMENT DE DONNÉES, PROGRAMME INFORMATIQUE ET SUPPORT LISIBLE PAR ORDINATEUR

(43) Date of publication of application: 27.08.2025
(73) Proprietor: Siemens Healthineers AG, 91301 Forchheim (DE); King's College London, London WC2R 2LS (GB)
(72) Inventor: Neji, Radhouene, London, N65XP (GB); Tripp, Donovan, London, SE1 7AL (GB); Botnar, Rene, London, SW19 8QW (GB); Kunze, Karl-Philipp, London, SW10 9JR (GB); Prieto, Claudia, London, SW19 8QW (GB)
(74) Representative: Siemens Healthineers Patent Attorneys

(56) References cited:
- US-A1- 2017 160 363
- DAVIES MIKE ET AL: "A Compressed Sensing Framework for Magnetic Resonance Fingerprinting", vol. 7, no. 4, 6 June 2014 (2014-06-06), pages 2623 - 2656, XP055797785, Retrieved from the Internet <URL:https://arxiv.org/pdf/1312.2465.pdf> DOI: 10.1137/130947246
- CLINE CHRISTOPHER C. ET AL: "AIR-MRF: Accelerated iterative reconstruction for magnetic resonance fingerprinting", MAGNETIC RESONANCE IMAGING, vol. 41, 14 July 2017 (2017-07-14), TARRYTOWN, NY, US, pages 29 - 40, XP055797763, ISSN: 0730-725X, DOI: 10.1016/j.mri.2017.07.007
- FANG ZHENGHAN ET AL: "Deep Learning for Fast and Spatially Constrained Tissue Quantification From Highly Accelerated Data in Magnetic Resonance Fingerprinting", IEEE TRANSACTIONS ON MEDICAL IMAGING, IEEE, USA, vol. 38, no. 10, 1 October 2019 (2019-10-01), pages 2364 - 2374, XP011748186, ISSN: 0278-0062, [retrieved on 20191001], DOI: 10.1109/TMI.2019.2899328
- MAHAPATRA D ET AL: "Integrating Segmentation Information for Improved MRF-Based Elastic Image Registration", IEEE TRANSACTIONS ON IMAGE PROCESSING, IEEE, USA, vol. 21, no. 1, 1 January 2012 (2012-01-01), pages 170 - 183, XP011411992, ISSN: 1057-7149, DOI: 10.1109/TIP.2011.2162738

## Description

The invention concerns a computer-implemented method for processing measurement information to select a respective one of multiple possible labels for multiple voxels or pixels of a one-dimensional or multidimensional medical image dataset. Additionally, the invention concerns a data processing system, a computer program, and a computer readable medium. Dictionary matching is a highly significant approach for implementing quantitative medical imaging, in particular to magnetic resonance imaging, since it allows multiple tissue parameters to be estimated even in the presence of complex interactions between these parameters. The quantitative determination of actual tissue parameters is highly relevant in medical imaging, since it can, e.g., allow for a more robust detection of diagnostically relevant features, e.g., due to a more robust tissue differentiation than, e.g., usual T1- or T2-weighted images, and can even allow the depiction of features that would otherwise be invisible. Dictionary matching can especially be used to generate parameter maps that describe the distribution of one or more specific parameters in the image dataset.

To perform dictionary matching in medical imaging, the matching is performed for each voxel or pixel of the medical image dataset separately. Since dictionary matching is a highly nonlinear process, even a small amount of noise in the measurement information can lead to a frequent local mislabeling and therefore, e.g., to a high noise floor in parameter maps that are generated based on dictionary matching. The resulting noise component can be reduced by postprocessing, e.g., by lowpass filtering or, alternatively, by spatial regularization, the latter approach being known e.g. from documents Davies Mike ET AL: "A Compressed Sensing Framework for Magnetic Resonance Fingerprinting", SIAM Journal on Imaging Sciences, vol. 7, no. 4, 6 June 2014 (2014-06-06), pages 2623-2656, DOI: 10.1137/130947246 and Cline Christopher C. ET AL: "AIR-MRF: Accelerated iterative reconstruction for magnetic resonance fingerprinting", MAGNETIC RESONANCE IMAGING, vol. 41, 14 July 2017 (2017-07-14), pages 29-40, TARRYTOWN, NY, US ISSN: 0730-725X, DOI: 10.1016/j.mri.2017.07.007. This, however, requires a trade-off between a lower noise floor on the one hand and a resolution and sharpness of the image on the other hand.

The invention is therefore based on the problem of improving the quality of quantitative maps in medical imaging, in particular when dictionary matching is used.

The problem is solved by a computer-implemented method for processing measurement information to select a respective one of multiple possible labels for multiple voxels or pixels of a one-dimensional or multidimensional medical image dataset based on reference information,
- wherein the reference information comprises a respective reference dataset comprising multiple reference values for each one of the possible labels,
- wherein each label is associated with a material parameter or a set of material parameters of a tissue that is potentially depicted in the medical image dataset,
- wherein the respective label for the respective voxel or pixel is selected by using a discrete optimization algorithm to optimize a cost function,
- wherein the cost function depends on
   - a first partial cost function comprising a respective measure for the difference between a measurement dataset for the respective voxel or pixel and the reference dataset assigned to the label assigned to this respective voxel or pixel, and
   - a second partial cost function comprising a respective difference measure for multiple selected pairs of mutually different voxels or pixels, wherein the respective difference measure is a measure for the difference between the material parameters or between the sets of material parameters associated with the labels selected for the voxels or pixels of the respective pair,
- wherein the measurement dataset comprises multiple measurement values that are or a single measurement value that is selected or determined from the measurement information for the respective voxel or pixel.

An optimization, especially a minimization, of the first partial cost function by itself could, e.g., be used to implement a labeling, using traditional dictionary matching. The incorporation of the second partial cost function into the cost function introduces a coupling between the labels at different voxels or pixels. By, e.g., including terms for all pairs of neighboring voxels or pixels or terms for all pairs of voxels or pixels that are within a given distance from each other, the second partial cost function can introduce a spatial regularization as an integral part of the optimization and therefore the labeling process. Such a regularization is highly efficient in suppressing the initially discussed artificial high-frequency variation in the labeling and can therefore, e.g., noticeably increases the image quality of parameter maps based on this labeling.

The feature, that the respective label is associated with a material parameter or a set of material parameters can on the one hand be understood to mean, that the label itself directly describes the material parameter or the set of material parameters. The label could however alternatively or additionally be associated with the material parameter or at least one of the material parameters by a look-up-table or other means of defining such an association.

The determination of an exact solution for a discrete optimization problem can be quite computationally intensive. Such an exact solution would typically still be possible with quite reasonable computational requirements, when only the first partial cost function and therefore a purely local dictionary matching would be used, since the number of possible solutions is limited to the number of possible labels in this case. It was found that a reasonably robust determination of labels concerning a single parameter is typically possible with a moderate number of labels and associated reference datasets that could also be identified as fingerprints. For a pure T₁-mapping it is typically sufficient to use less than 1000 different labels and associated fingerprints. For a pure T₂-mapping a reasonable mapping quality can be reached with less than 200 different labels and associated fingerprints. For determining multiple material parameters at the same time, preferably more than 1000 or even more than 10,000 different labels and associated fingerprints are used. A very high precision is typically reached in this case, once the number of labels and associated fingerprints reaches approximately 200,000.

The determination of an exact solution becomes a lot more computationally intensive due to the non-locality introduced by the second partial cost function. Even when only pairs of neighboring voxels or pixels are taken into account in the second partial cost function, this can lead to an indirect coupling of the solution for any given pixels or voxel to the solutions for all other voxels or pixels. Therefore, when the number of labels is n and the number of voxels or pixels in the medical image dataset is m, n^{m} possible solutions would need to be considered for an exact solution. Therefore, it does seem to be counterintuitive at first to take the second partial cost function into account during the optimization. As discussed in more detail below, it was however found, that a good approximate solution for the full cost function can still be determined with reasonable computational complexity.

When describing the assignment of the respective label to the respective voxel or pixel by using integers, the optimization, in particular the minimization or maximization, of the cost function can be considered as an integer programming problem. While an exact solution to integer programming problems is computationally intensive, a multitude of suitable approaches are known, to iteratively determine approximate iterative solutions of such optimization problems.

The cost function used in the method according to the present invention is especially suitable for such approaches, since, as discussed later, this cost function has the same structure as the energy of a discrete Markov random field (MRF). The respective voxel or pixel in the medical image dataset can then be considered as a vertex of the graph forming the MRF. Each one of the selected pairs of mutually different voxels or pixels then corresponds to a respective edge of the MRF, and the potential functions of the MRF is identified with the difference measure.

Therefore, any of the many discrete optimization algorithms that are usable to optimize a MRF can also be used to optimize the full cost function in the method according to the present invention. Examples for such optimization algorithms are algorithms based on graph-cuts, e.g., the α-expansion or the αβ-swap, or on belief propagation, e.g., loopy belief propagation or tree reweighted message-passing. In particular the PD1, PD2 or PD3 (PD: prime-dual) algorithms and their various variants disclosed in N. Komodakis and G. Tziritas, "Approximate labeling via graph cuts based on linear programming," IEEE transactions on pattern analysis and machine intelligence, vol. 29, no. 8, pp. 1436-1453, 2007 (referred to as Komodakis (1) in the further text), or the fast prime-dual MRF optimization disclosed in N. Komodakis, G. Tziritas, and N. Paragios, "Performance vs computational efficiency for optimizing single and dynamic MRFs: Setting the state of the art with primal-dual strategies," Computer Vision and Image Understanding, vol. 112, no. 1, pp. 14-29, 2008 (referred to as Komodakis (2) in the further text), could be used as optimization algorithms.

The cost function can, e.g., be written as: ${\sum }_{p ∈ I} D \left(\hat{f}\left(p\right),\hat{s}\left({θ}_{1}\left(p\right),…,{θ}_{N}\left(p\right)\right)\right) + {\sum }_{\left(p, q\right) ∈ A} {w}_{pq} {\sum }_{i = 1}^{N} {d}_{i} \left({θ}_{i}\left(p\right),{θ}_{i}\left(q\right)\right)$

The sum over the measure for the difference D over the individual voxels or pixels p in the medical image dataset I corresponds to the first partial cost function. ***f̂***(*p*) corresponds to the actual measurement dataset for the respective voxel or pixel p and ***ŝ***(*θ*₁(*p*), *... , θ_{N}*(*p*)) corresponds to the reference dataset assigned to the label assigned to this respective voxel or pixel p. To show the interplay between the partial cost functions more clearly, the label itself is not explicitly referred to in the exemplary cost function given above. Instead, the label is identified by its associated set of material parameters *θ*₁(*p*), *... , **θ**_{N}*(*p*)*.*

The second partial cost function is a sum over the selected pairs (p, q) of mutually different voxels or pixels p, q over the group A of the selected pairs. The individual summand of this outer sum corresponds to the difference measure for the difference between the different sets of the material parameters ***θ**ᵢ* associated with the different labels selected for the different voxels or pixels p, q of the respective pair, wherein the difference measure is written as a sum of partial difference measures dᵢ for each one of the material parameters ***θᵢ***. The weighting factor w_{pq} can be one in the simplest case. By scaling the weighting factor for all pairs, the strength of the spatial regularization can be adjusted. Using different weighting factors for different pairs can, e.g., allow for a different weighting of different pairs to, e.g., apply a regularization that takes indirect neighbors into account, but to a lesser degree than direct neighbors, similar to the use of an extended filter kernel. It should be noted, that the partial difference measures dᵢ could be different from each other, e.g., to allow for a different weighting for the differences for different material parameters.

The energy of a discrete MRF can be written as (see, e.g., equation (1) in Komodakis (2)): ${\sum }_{p ∈ V} {c}_{p} \left({x}_{p}\right) + {\sum }_{\left(p, q\right) ∈ E} {w}_{pq} d \left({x}_{p}, {x}_{q}\right)$

Wherein V is the Group of all Vertices, E is the group of all edges, *cₚ*(*xₚ*) is the cost of assigning the label xₚ to the vertex p and *d*(*xₚ,x_{q}*) is he potential function. By considering each voxel or pixel as a vertex of the MRF and each selected pair of mutually different voxels or pixels as an edge of the MRF and by setting *cₚ*(*xₚ*) *=* D(***f̂***(*p*),***ŝ***(*θ*₁(*p*), *... , θ_{N}*(*p*))) and $d \left({x}_{p}, {x}_{q}\right) = {\sum }_{i = 1}^{N} {d}_{i} \left({θ}_{i}\left(p\right),{θ}_{i}\left(q\right)\right)$ the cost function can be treated as the energy of the discrete MRF and optimized accordingly.

The use of an iterative approximate solution to the optimization problem also has an additional advantage. The discrete nature of a dictionary matching normally presents a challenge to the application of spatial regularization techniques common in image reconstruction techniques, e.g., in iterative reconstructions in magnetic resonance imaging, since such iterative methods typically require a differentiable cost function for the reconstruction to, e.g., allow the use of a gradient descent algorithm. Many of the mentioned discrete optimization algorithms do however use an optimization of continuous variables to find an approximate solution for the discrete optimization. Such algorithms are well-suited to be used in conjunction with other approaches for iterative optimization of the medical image dataset, especially with iterative reconstruction methods.

Appropriate distance functions to determine the respective measure for the difference between the respective measurement dataset and the respective reference dataset and/or for determining the respective difference measure for the respective selected pair of pixels or voxels are, e.g., the Euclidean distance or the cosine distance. For normalized reference datasets, these will define the same optimum.

The labeling can in particular be used to generate a parameter map for at least one material parameter based on the labels assigned to the voxels or pixels. After the labeling is complete, it is, e.g., possible to generate a parameter map directly by assigning the value of the material parameter or of one on the material parameters of the set of material parameters associated to the respective label to the respective voxel or pixel. Alternatively or additionally, derived parameter maps can be generated by determining the value of the parameter map at the respective voxel or pixel based on multiple material parameters of the respective material parameter set and/or by taking at least one additional parameter, e.g., a global parameter like the weight and/or the gender of a patient, into account.

In particular, the medical image dataset can be a two-dimensional, three-dimensional or four-dimensional medical image dataset. The multiple measurement values or the single measurement value can be explicitly or implicitly specified by the measurement information. It is, e.g., possible that the measurement information comprises one or multiple magnetic resonance signals that are first reconstructed to specify at respective measurement value for multiple points in time for each voxel or pixel of the medical image dataset. In most use cases, the different measurement values for the respective voxel or pixel will concern the same type of measurement data, e.g., a magnetic resonance signal. In principle, it would however also be possible to provide data from multiple imaging methods, e.g., from a combined magnetic resonance tomography and an x-ray based computer tomography registered to the magnetic resonance tomography to provide different measurement values for the respective measurement dataset.

The number of measurement values in each measurement dataset is preferably identical to the number of reference values in each of the reference datasets. The reference dataset can, e.g., be obtained by a simulations of known acquisition physics, for example based on Bloch simulations or extended phase graph simulations of magnetic resonance imaging sequences. Alternatively or additionally, it can be based on analytical equations based on the know physics of the measurements.

While the use of multiple measurement values in the measurement dataset that are selected or determined from the measurement information for the respective voxel or pixel can improve the robustness of the dictionary matching, in some use cases it can be sufficient to use a measurement dataset only comprising a single measurement value. The measurement information can be acquired outside of the claimed method. The measurement information can, e.g., be received from a database, a server, an internal memory of a medical imaging device or from the internal memory of or from a different subroutine running on a data processing system implementing the claimed method. Alternatively, the measurement information can be acquired by performing the relevant measurements as a step of the claimed computer-implemented method.

Each of the voxels or pixels of the medical image dataset, can be part of at least one of the selected pairs of voxels or pixels that also comprises a further one of the voxels or pixels that is adjacent to that voxel or pixel. By taking pairs of adjacent voxels or pixels into account in the second partial cost function, a spatial regularization can be applied to the labeling of the medical image dataset. This can especially reduce high-frequency noise, while avoiding or reducing a blurring of the image and an elimination of fine detail, that would result from a filtering or a similar postprocessing that could be used to lower in the noise floor.

When the medical image dataset is an at least two-dimensional medical image dataset, each of the voxels or pixels of the medical image dataset can in particular be at least part of one or two of the selected pairs of voxels or pixels, which also comprises a further one of the voxels or pixels that is adjacent to that voxel or pixel in the respective dimension, for each of the dimensions of the medical image dataset. In particular each one of the voxels or pixels can share a respective selected pair with each one of its neighbors in each one of the dimensions.

Therefore, a pixel in a two-dimensional medical image dataset that is not located at the edge or corner of the medical image dataset can at least be a member of four selected pairs that it shares with its neighbors. A voxel in a three-dimensional medical image dataset that is not located at the edge, corner, or outer surface of the medical image dataset, can at least be a member of six selected pairs that it shares with its neighbors and a voxel in a four-dimensional medical image dataset that is not located at the edge, corner, or outer surface of the medical image dataset can at least be a member of eight selected pairs, that it shares with its neighbors.

The selected pairs can be explicitly defined in the algorithm or, e.g., be defined by a kind of convolution kernel or filter kernel that describes, which of the close by voxels or pixels should be paired with each of one of the voxels or pixels.

It should be noted, that different weights can be assigned to the different measures for different selected pairs in the cost function. Such weights can especially be defined by a convolution kernel or filter kernel, that defines the spatial regularization and therefore how strongly the label of a respective pixel or voxel is influenced by the labels of the different voxels or pixels in the neighborhood surrounding the respective pixel or voxel.

In a preferred embodiment, the measurement information can be based on a magnetic resonance measurement sequence. Measurement information based on a magnetic resonance sequence, in particular on a magnetic resonance tomography sequence, is especially suitable to depicted material contrast, e.g., a contrast between different tissues and/or between different fluids within a patient and/or between fluid and tissue, based on magnetic resonance fingerprinting or other dictionary matching techniques. The inventive method could however, e.g., also be used to evaluate X-ray, computer tomography or emission tomography data, wherein the different measurement values of the respective measurement dataset could, e.g., correspond to the intensities at different photon energies.

The material parameter can be or the set of material parameters can comprise at least one of: a T₁-time and/or a T₂-time and/or a spin density and/or a measure for the amount of a specific tissue and/or fluid in the respective voxel or pixel. The mentioned material parameters can be highly relevant when evaluating the medical image dataset and are well suited for a determination using magnetic resonance fingerprinting or related techniques based on dictionary matching.

The magnetic resonance measurement sequence can comprise a sequence of a partial sequences, wherein the measurement information comprises multiple preliminary medical image datasets or wherein multiple preliminary medical image datasets are determined based on the measurement information, wherein each one of the preliminary medical image datasets is based on partial measurement information acquired during a respective partial sequence, wherein each one of the preliminary medical image datasets provides a respective one of the multiple measurement values for the respective measurement set, wherein the partial sequences differ from each other concerning the number and/or order and/or types of excitation pulses used during the respective partial sequence and/or concerning the timing of the excitation pulses during the respective partial sequence.

By varying the number and/or order and/or types and/or timing of the used excitation pulses between the different partial sequences, the measurement values provided by the different preliminary medical image datasets for the respective voxel or pixel can, e.g., depend on different combinations of the various material parameters.

It is, e.g., possible that one of the preliminary medical image datasets is strongly dependent on T₁-times and weakly dependent on T₂-times, while a second one of the preliminary medical image datasets is weakly dependent on T₁-times and strongly dependent on T₂-times. Even in cases, wherein both of the preliminary medical image datasets additionally depend on one or more further material parameters, e.g., on a local a spin density, the discussed labeling based on dictionary matching can be used to, e.g., determine parameter maps for the T₁- and T₂-times that are at least approximately independent of from each other and the additional material parameter or parameters. To improve the robustness of the labeling and therefore of the determination of the respective local value of the respective material parameter and/or to allow for the determination of a larger number of material parameters, the use of more than two partial sequences is typically advantageous.

The magnetic resonance measurement sequence can comprise multiple different partial sequences, wherein the different partial sequences could be used independently during a medical imaging to the provide different types of contrast, e.g., different degrees of T₁- or T₂-weighting. It is however also possible, to use a pseudo-random variation, e.g., of the flip-angles and/or of a used to timing, to generate a fingerprint of the respective tissue depicted in the respective voxel or pixel that is characteristic for this tissue and the used pseudo-random sequence.

The number of pulses and/or of their order can, e.g., be varied to use different types of imaging sequences, e.g., to use a spin echo sequence in some of the partial sequences and an inversion recovery sequence in the other partial sequences. Additionally, the number of pulses in the partial sequence can be varied to, e.g., use a T₂-preperation pulse in only some of the partial sequences. This approach and/or a variation of the timing within the respective sequence can, e.g., be used to achieve different degrees of T₁- and/or T₂-weighting in the different partial sequences.

Different types of excitation pulses can especially be excitation pulses with different flip-angles. A variation of the pulse type between partial sequences can in particular be used provide different sequence types as partial sequences, as discussed above, and/or to apply, e.g., relatively small pseudo-random variations of the flip angles between the different partial sequences for a pseudo-random fingerprinting.

Each part of the magnetic resonance measurement sequence can in principle be considered as a separate medical imaging sequence providing a separate preliminary medical image dataset. It is however, e.g., possible, to use a relatively short waiting time between the different partial sequences, therefore allowing for an influence of the excitation pulses during the previous partial sequence on the preliminary medical image dataset acquired during the current partial sequence.

The magnetic resonance measurement sequence can be designed to provide measurement information suitable for an evaluation using magnetic resonance fingerprinting or a different dictionary matching technique. Additionally or alternatively, the selection of the respective label for the respective voxel or pixel can be based on magnetic resonance fingerprinting or the or a different dictionary matching technique. Details and advantages of dictionary matching techniques and in particular of fingerprinting techniques where already discussed above.

The reference dataset can be based on magnetic resonance pulse sequence simulations and/or equations describing signal evolutions in magnetic resonance imaging. The simulation can be performed using, e.g., Bloch simulations or Extended Phase Graph simulations. Both approaches are well known in the prior art. The signal evolution in magnetic resonance imaging can, in particular, be described using the well-known Bloch equations. While these can be used to determine approximate solutions in the context of a simulation of a magnetic resonance imaging, in many use cases it is also possible to find an exact solution.

In general, the reference dataset can be based on reference measurements and/or on a simulation of an imaging technique, on which the measurement information is based, and/or on a solution of analytical equations describing the physics of that imaging technique. It is, e.g., possible to perform reference measurements on one or multiple phantoms with a known distribution of material parameters to determine the reference data. Examples for the use of simulations and the solving of analytical equations to determine the reference dataset where already given above.

The discrete optimization algorithm can be based on a primal-dual optimization approach. The following example of such an algorithm is based on the detailed discussion of the primal-dual schema in section 3. of Komodakis (2). For simplicities' sake, it is assumed, that the cost function is to be minimized and that it was already rewritten in the form of the energy of a discrete MRF, as discussed above. The approach can obviously be easily modified to allow for a maximization of the cost function and can also be directly applied to the cost function without an explicit rewrite.

The MRF energy that is to be minimized can be essentially linearized by introducing two types of indicator variables. The indicator variables xₚ(a) are used for indicating, which label is assigned to each MRF node. If xₚ(a) *=* 1, label a is assigned to p and otherwise xₚ(a) can be zero. The indicator variables x_{pq}(a,b) are used to indicate, which pair of labels is assigned to each pair of neighboring nodes (a,b). If x_{pq}(a,b) is 1, the label a is assigned to p and the label b is assigned to q. The primal problem is then to minimize the function ${\sum }_{p ∈ V} {\sum }_{a ∈ L} {c}_{p} \left(a\right) {x}_{p} \left(a\right) + {\sum }_{\left(p, q\right) ∈ E} {w}_{pq} \left({\sum }_{a , b ∈ L} d \left(a, b\right) {x}_{pq} \left(a, b\right)\right) .$

The variables xₚ, x_{pq} are used as the primal variables during the optimization.

According to Komodakis (2) the dual problem can then be a maximation problem with the dual variables y_{pq}(a)=-y_{qp}(a) for each label a and edge (q, p) and the problem can be reformulated using the auxiliary variables ${h}_{p} \left(a\right) = {c}_{p} \left(a\right) + {\sum }_{\left(p, q\right) ∈ E} {y}_{qp} \left(a\right)$ for each vertex p and label a and using the notation xₚ to indicate the active label at the vertex p to read: ${h}_{p} \left({x}_{p}\right) = {min}_{a ∈ L} {h}_{p} \left(a\right)$ ${y}_{pq} \left({x}_{p}\right) + {y}_{qp} \left({x}_{q}\right) = {w}_{pq} d \left({x}_{p}, {x}_{q}\right)$ ${y}_{pq} \left(a\right) + {y}_{qp} \left(b\right) \leq 2 {w}_{pq} {d}_{max}$

The constant dₘₐₓ is the maximum value that d(a,b) can reach for an arbitrary pair of labels a, b. The optimization alternates between updating the dual variables y_{pq} and y_{qp} and therefore the height hₚ(a) of the respective label at the respective vertex, using the previously stated conditions, and selecting the active label for the respective vertex p. The update of the dual variables can be performed individually for each one of the labels and can, e.g., be performed by solving a max-flow problem in a capacitated graph defined by these variables, as discussed in detail in section 4 of Komodakis (2). A detailed derivation of the discussed approach is, e.g., given in Komodakis (1).

Besides the computer-implemented method, the invention also concerns a data processing system, characterized in that the data processing system is configured to carry out the computer-implemented method for processing measurement information according to the present invention.

Additionally, the invention concerns a data processing system, that is designed to carry out the previously discussed computer-implemented method. The data processing system can, e.g., be a desktop computer, a server or be implemented as a cloud solution. Additionally or alternatively, it can be integrated into a medical imaging device. The data processing system can have hardware and/or software interfaces for receiving the various inputs discussed above.

The invention also concerns a computer program that comprises instructions to carry out the previously discussed computer-implemented method when the program is executed on a data processing system.

Additionally, the invention concerns a computer-readable medium comprising this computer program.

Other objects and features of the present invention will become apparent from the following detailed description considered in conjunction with the accompanying drawings. The drawings, however, are only principle sketches designed solely for the purpose of illustration and do not limit the invention. The drawings show:
Fig. 1 an overview of relevant features and data structures in an exemplary embodiment of the computer-implemented method for processing measurement information according to the present invention,
Fig. 2 a flow chart of the embodiment according to fig. 1, and
Fig. 3 an exemplary embodiment of the data processing system according to the present invention.

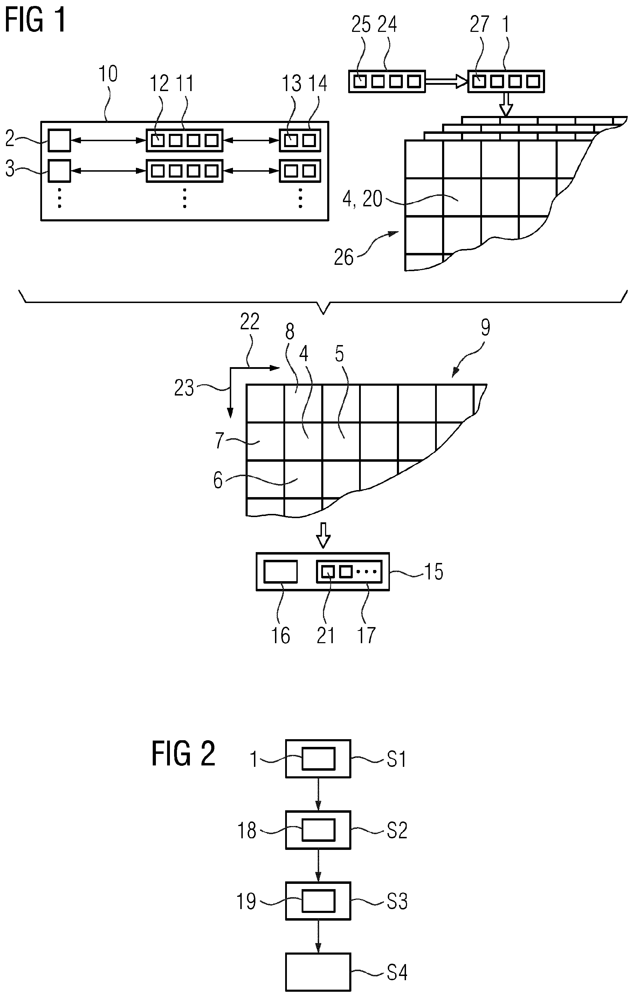

Fig. 1 gives an overview of relevant features and data structures of a computer-implemented method for processing measurement information 1 to select a respective one of multiple possible labels 2, 3 for the pixels 4-8 of a two-dimensional medical image dataset 9 based on reference information 10. As already discussed in the general part of the description, the discussed method could easily be modified to label a medical image dataset with a single dimension or with more than 2 dimensions. In the latter case, the labels 2, 3 would be applied to voxels instead of pixels 4-8.

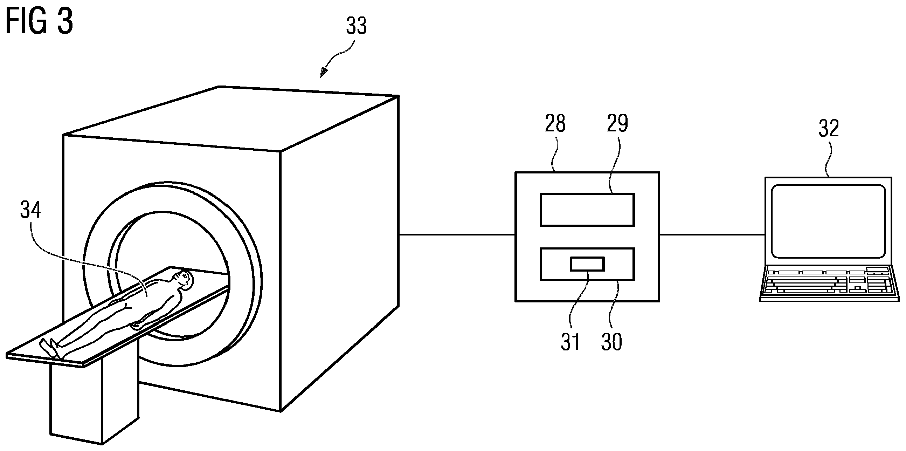

The method will be discussed in conjunction with fig. 2 that shows a flowchart of the method, and fig. 3 that shows a possible use case for the method and a data processing system 28 implementing the method.

In step S1 measurement information 1 is received. In the example shown in figure 3, the measurement information 1 is received by the data processing system 28 from a medical imaging device 33. The measurement information 1 can be received immediately after its acquisition by imaging the patient 34 using the medical imaging device 33 or it could, e.g., be stored and received at a later point in time.

In the example, the measurement information 1 is based on a magnetic resonance measurement sequence 24, that comprises a sequence of a partial sequences 25. In the example, four partial sequences 25 are used, that acquire respective partial measurement information 27 concerning the same volume by using different magnetic preparations, e.g., inversion recovery and T2 preparation pulses, respectively. As already discussed in the general part of the description, it would, e.g., alternatively be possible to use a pseudo-random variation of acquisition parameters between the partial sequences 25.

Based on each partial measurement information 27, a respective preliminary medical image datasets 26 can then be reconstructed. Since four medical image datasets 26 are reconstructed and each one of the medical image datasets 26 provides a separate measurement value 20 for each pixel 4-8, a measurement dataset 18 comprising four separate measurement values 20 can then be provided for each of the pixels 4-9 of the medical image dataset 9 in step S2.

In step S3, a respective label 2, 3 is selected for the respective pixel 4-8 by using a discrete optimization algorithm 19 to optimize a cost function 15. The respective label 2, 3 is defined by the reference information 10 that comprises a respective reference dataset 11 comprising multiple reference values 12 for each one of the possible labels 2, 3. Additionally the reference information 10 assigns a set 14 of material parameters 13 to each one of the labels. In the example, the two material parameters 13 define a T₁-time and T₂-time associated with the respective label.

The reference information 10 therefore defines a correlation between the material parameters 13 and a fingerprint defined by the reference information 10. It should be noted, that the label could be directly defined by the material parameters 10 and does not need to be a distinct information.

In the example, 1339 labels that correspond to 103 × 103 combinations of a T₁-time and a T₂-time are used. The reference values 12 can, e.g., be obtained via 1D Bloch simulations. The T₁- and T₂-times can be logarithmically spaced in intervals of 80 ms to 1600 ms and of 16 ms to 320 ms respectively.

The cost function 15 is the sum of two partial cost functions 16, 17. The first partial cost function 16 comprises a respective measure for the difference between the measurement dataset 18 for the respective pixel 4-8 and therefore the fingerprint measured for that pixel and the reference dataset 11 assigned to the label 2, 3 assigned to this pixel 4-8. The minimization of the partial cost function 15 by itself would correspond to a separate dictionary matching for each individual pixel 4-8 without any consideration of the spatial proximity between the pixels 4-8. Such a purely local optimization usually results in a spatially unstable result due to the potentially complex topology of the dictionary, translating into noisy parameter maps.

The second partial cost function 17 comprising a respective difference measure for multiple selected pairs 21 of adjacent pixels 4-8. In the example the pixel 4 would, e.g., be a member of two selected pairs 21 of pixels for each one of the dimensions 22, 23 of the medical image dataset 9, namely of the pairs (4, 5), (4, 6), (4, 7) and (4, 8).

The second partial cost function 17 can comprise separate summands for each selected pair 21 of pixels 4-8 and each one of the material parameters 13. Different weighting factors can be used for the summands for the different material parameters 13. In an exemplary embodiment, good results were achieved with the weight w₁ = 5*10⁻⁶ for summands concerning T₁-times and the weight w₂ = 1.5*10⁻⁵ for summands concerning T₂-times respectively. The individual summand can be determined by taking the absolute value of the difference of the values of the respective material parameters 13 between the pixels 4-8 in the pair 21.

The cost function is minimized in the example by using the fast prime-dual MRF optimization disclosed in Komodakis (2) that was already discussed in the general part of the description. The cosine distance is used as the measure for the difference between a measurement dataset 18 for the respective pixel 4-8 and the reference dataset 11 assigned to the label 2, 3 assigned to the respective pixel 4-8.

Once this optimization is concluded, a respective parameter map is generated for each one of the material parameters 13 in step S4, based on the respective label 2, 3 assigned to the respective pixel 4 to 8. In an alternate embodiment, the parameter map could be directly created by the assignment of the respective label 2, 3 to the respective pixel 4 to 8, e.g., when the value of the label 2, 3 directly corresponds to the material parameter 13 or comprises a concatenation of the material parameters 13 of the set 14.

The exemplary embodiment therefore generates accurate and spatially regulated T₁- and T₂-maps. Compared to parameter maps that are based on purely local fingerprinting, e.g., on a minimization of the first partial cost function by itself, a notably lower high frequency noise was observed in phantom measurements as well as in in-situ measurements, e.g., when imaging the liver of a patient.

As shown in fig. 3, the data processing system 28 can be implemented by executing a computer program 31 on a programmable processor 29. The computer program 31 can, e.g., be stored in an internal memory 30 of the data processing system 28. The generated parameter maps can, e.g., be output to the personal computer 32.

In the example the data processing system 28 is designed as a separate device and could, e.g., be implemented as a server or as a cloud solution. It would, however, also be possible, to integrate the processing system 28 into the medical imaging device 33 or into the personal computer 32.

Independent of the grammatical term usage, individuals with male, female or other gender identities are included within the term.

## Claims

1. Computer-implemented method for processing measurement information (1) to select a respective one of multiple possible labels (2, 3) for multiple voxels or pixels (4-8) of a one-dimensional or multidimensional medical image dataset (9) based on reference information (10),
- wherein the reference information (10) comprises a respective reference dataset (11) comprising multiple reference values (12) for each one of the possible labels (2, 3),
- wherein each label (2, 3) is associated with a material parameter (13) or a set (14) of material parameters (13) of a tissue that is potentially depicted in the medical image dataset (9),
- wherein the respective label (2, 3) for the respective voxel or pixel (4-8) is selected by using a discrete optimization algorithm (19) to optimize a cost function (15),
- wherein the cost function (15) depends on
• a first partial cost function (16) comprising a respective measure for the difference between a measurement dataset (18) for the respective voxel or pixel (4-8) and the reference dataset (11) assigned to the label (2, 3) assigned to this respective voxel or pixel (4-8), and
• a second partial cost function (17) comprising a respective difference measure for multiple selected pairs (21) of mutually different voxels or pixels (4-8), wherein the respective difference measure is a measure for the difference between the material parameters (13) or between the sets (14) of material parameters (13) associated with the labels (2, 3) selected for the voxels or pixels (4-8) of the respective pair (21),
- wherein the measurement dataset (18) comprises multiple measurement values (20) that are or a single measurement value (20) that is selected or determined from the measurement information (1) for the respective voxel or pixel (4-8).

2. Computer-implemented method according to claim 1, **characterized in that** each of the voxels or pixels (4-8) of the medical image dataset (9), is part of at least one of the selected pairs (21) of voxels or pixels (4-8) that also comprises a further one of the voxels or pixels (4-8) that is adjacent to that voxel or pixel (4-8).

3. Computer-implemented method according to claim 1 or 2, wherein the medical image dataset (9) is an at least two-dimensional medical image dataset (9), , **characterized in that** each of the voxels or pixels (4-8) of the medical image dataset (9) is at least part of one or two of the selected pairs (21) of voxels or pixels (4-8), which also comprises a further one of the voxels or pixels (4-8) that is adjacent to that voxel or pixel (4-8) in the respective dimension (22, 23), for each of the dimensions (22, 23) of the medical image dataset (9).

4. Computer-implemented method according to one of the preceding claims, **characterized in that** the measurement information (1) is based on a magnetic resonance measurement sequence (24).

5. Computer-implemented method according to claim 4, wherein the material parameter (13) is or the set (14) of material parameters (13) comprises at least one of: a T₁-time and/or a T₂-time and/or a spin density and/or a measure for the amount of a specific tissue and/or fluid in the respective voxel or pixel (4-8).

6. Computer-implemented method according to claim 4 or 5, **characterized in that** the magnetic resonance measurement sequence (24) comprises a sequence of a partial sequences (25), wherein the measurement information (1) comprises multiple preliminary medical image datasets (26) or wherein multiple preliminary medical image datasets (26) are determined based on the measurement information (1), wherein each one of the preliminary medical image datasets (26) is based on partial measurement information (27) acquired during a respective partial sequence (25), wherein each one of the preliminary medical image datasets (26) provides a respective one of the multiple measurement values (20) for the respective measurement set (18), wherein the partial sequences (25) differ from each other concerning the number and/or order and/or types of excitation pulses used during the respective partial sequence (25) and/or concerning the timing of the excitation pulses during the respective partial sequence (25).

7. Computer-implemented method according to one of the claims 4 to 6, **characterized in that** the magnetic resonance measurement sequence (24) is designed to provide measurement information (1) suitable for an evaluation using magnetic resonance fingerprinting or a different dictionary matching technique and/or **in that** the selection of the respective label (2, 3) for the respective voxel or pixel (4-8) is based on magnetic resonance fingerprinting or the or a different dictionary matching technique.

8. Computer-implemented method according to one of the claims 4 to 7, **characterized in that** the reference dataset (11) is based on magnetic resonance pulse sequence simulations and/or equations describing signal evolutions in magnetic resonance imaging.

9. Computer-implemented method according to one of the preceding claims, **characterized in that** the reference dataset (11) is based on reference measurements and/or on a simulation of an imaging technique (1), on which the measurement information (1) is based, and/or on a solution of analytical equations describing the physics of that imaging technique.

10. Computer-implemented method according to one of the preceding claims, **characterized in that** the discrete optimization algorithm (19) is based on a primal-dual optimization approach.

11. Data processing system, **characterized in that** it is configured to carry out the computer-implemented method according to one of the preceding claims.

12. Computer program, **characterized in that** it comprises instructions to carry out the computer-implemented method according to one of the claims 1 to 7 when the program is executed on a data processing system (28).

13. Computer-readable medium comprising the computer program (31) according to claim 12.

## Patentansprüche

1. Computerimplementiertes Verfahren zum Verarbeiten von Messinformationen (1) zum Auswählen eines jeweiligen von mehreren möglichen Etiketten (2, 3) für mehrere Voxel oder Pixel (4-8) eines eindimensionalen oder mehrdimensionalen medizinischen Bilddatensatzes (9) basierend auf Referenzinformationen (10),
- wobei die Referenzinformationen (10) einen jeweiligen Referenzdatensatz (11) umfassen, der mehrere Referenzwerte (12) für jedes der möglichen Etiketten (2, 3) umfasst,
- wobei jedes Etikett 2, 3) einem Materialparameter (13) oder einem Satz (14) von Materialparametern (13) eines Gewebes zugeordnet ist, das potenziell in dem medizinischen Bilddatensatz (9) dargestellt ist,
- wobei das jeweilige Etikett (2, 3) für das jeweilige Voxel oder Pixel (4-8) unter Verwendung eines diskreten Optimierungsalgorithmus (19) ausgewählt wird, um eine Kostenfunktion (15) zu optimieren,
- wobei die Kostenfunktion (15) von Folgendem abhängt:
• einer ersten Teilkostenfunktion (16), die ein jeweiliges Maß für die Differenz zwischen einem Messdatensatz (18) für das jeweilige Voxel oder Pixel (4-8) und dem Referenzdatensatz (11), der dem diesem jeweiligen Voxel oder Pixel (4-8) zugewiesenen Etikett (2, 3) zugewiesen ist, umfasst, und
• einer zweiten Teilkostenfunktion (17), die ein jeweiliges Differenzmaß für mehrere ausgewählte Paare (21) voneinander verschiedener Voxel oder Pixel (4-8) umfasst, wobei das jeweilige Differenzmaß ein Maß für die Differenz zwischen den Materialparametern (13) oder zwischen den Sätzen (14) von Materialparametern (13) ist, die den für die Voxel oder Pixel (4-8) des jeweiligen Paars (21) ausgewählten Etiketten (2, 3) zugeordnet sind,
- wobei der Messdatensatz (18) mehrere Messwerte (20) oder einen einzelnen Messwert (20), die/der aus den Messinformationen (1) für das jeweilige Voxel oder Pixel (4-8) ausgewählt oder bestimmt werden/wird, umfasst.

2. Computerimplementiertes Verfahren nach Anspruch 1, **dadurch gekennzeichnet, dass** jedes der Voxel oder Pixel (4-8) des medizinischen Bilddatensatzes (9) Teil mindestens eines der ausgewählten Paare (21) von Voxeln oder Pixeln (4-8) ist, das auch ein weiteres der Voxel oder Pixel (4-8) umfasst, das zu diesem Voxel oder Pixel (4-8) benachbart ist.

3. Computerimplementiertes Verfahren nach Anspruch 1 oder 2, wobei der medizinische Bilddatensatz (9) ein mindestens zweidimensionaler medizinischer Bilddatensatz (9) ist, **dadurch gekennzeichnet, dass** jedes der Voxel oder Pixel (4-8) des medizinischen Bilddatensatzes (9) für jede der Dimensionen (22, 23) des medizinischen Bilddatensatzes (9) mindestens Teil eines oder zweier der ausgewählten Paare (21) von Voxeln oder Pixeln (4-8) ist, das auch ein weiteres der Voxel oder Pixel (4-8) umfasst, das zu diesem Voxel oder Pixel (4-8) in der jeweiligen Dimension (22, 23) benachbart ist.

4. Computerimplementiertes Verfahren nach einem der vorhergehenden Ansprüche, **dadurch gekennzeichnet, dass** die Messinformationen (1) auf einer Magnetresonanzmesssequenz (24) basieren.

5. Computerimplementiertes Verfahren nach Anspruch 4,
wobei der Materialparameter (13) mindestens einer von folgenden ist oder der Satz (14) von Materialparametern (13) mindestens einen von folgenden umfasst: eine T₁-Zeit und/oder eine T₂-Zeit und/oder eine Spindichte und/oder ein Maß für die Menge eines spezifischen Gewebes und/oder Fluids in dem jeweiligen Voxel oder Pixel (4-8).

6. Computerimplementiertes Verfahren nach Anspruch 4 oder 5, **dadurch gekennzeichnet, dass** die Magnetresonanzmesssequenz (24) eine Sequenz von Teilsequenzen (25) umfasst, wobei die Messinformationen (1) mehrere vorläufige medizinische Bilddatensätze (26) umfassen oder wobei mehrere vorläufige medizinische Bilddatensätze (26) basierend auf den Messinformationen (1) bestimmt werden, wobei jeder der vorläufigen medizinischen Bilddatensätze (26) auf Teilmessinformationen (27) basiert, die während einer jeweiligen Teilsequenz (25) erfasst werden, wobei jeder der vorläufigen medizinischen Bilddatensätze (26) einen jeweiligen der mehreren Messwerte (20) für den jeweiligen Messsatz (18) bereitstellt, wobei sich die Teilsequenzen (25) hinsichtlich der Anzahl und/oder Reihenfolge und/oder Arten von Anregungsimpulsen, die während der jeweiligen Teilsequenz (25) verwendet werden, und/oder hinsichtlich der Zeitvorgabe der Anregungsimpulse während der jeweiligen Teilsequenz (25) voneinander unterscheiden.

7. Computerimplementiertes Verfahren nach einem der Ansprüche 4 bis 6, **dadurch gekennzeichnet, dass** die Magnetresonanzmesssequenz (24) dazu ausgelegt ist, Messinformationen (1) bereitzustellen, die für eine Auswertung mittels Magnetresonanzfingerabdruckverfahren oder einer anderen Nachschlagewerkabgleichstechnik geeignet sind, und/oder dass die Auswahl des jeweiligen Etiketts (2, 3) für das jeweilige Voxel oder Pixel (4-8) auf Magnetresonanzfingerabdruckverfahren oder der oder einer anderen Nachschlagewerkabgleichstechnik basiert.

8. Computerimplementiertes Verfahren nach einem der Ansprüche 4 bis 7, **dadurch gekennzeichnet, dass** der Referenzdatensatz (11) auf Magnetresonanzimpulssequenzsimulationen und/oder Gleichungen, die Signalevolutionen in der Magnetresonanztomographie beschreiben, basiert.

9. Computerimplementiertes Verfahren nach einem der vorhergehenden Ansprüche, **dadurch gekennzeichnet, dass** der Referenzdatensatz (11) auf Referenzmessungen und/oder auf einer Simulation einer Bildgebungstechnik (1), auf der die Messinformationen (1) basieren, und/oder auf einer Lösung analytischer Gleichungen, die die Physik dieser Bildgebungstechnik beschreiben, basiert.

10. Computerimplementiertes Verfahren nach einem der vorhergehenden Ansprüche, **dadurch gekennzeichnet, dass** der diskrete Optimierungsalgorithmus (19) auf einem Primal-dual-Optimierungsansatz basiert.

11. Datenverarbeitungssystem, **dadurch gekennzeichnet, dass** es zum Ausführen des computerimplementierten Verfahrens nach einem der vorhergehenden Ansprüche ausgelegt ist.

12. Computerprogramm, **dadurch gekennzeichnet, dass** es Befehle zum Ausführen des computerimplementierten Verfahrens nach einem der Ansprüche 1 bis 7 umfasst, wenn das Programm auf einem Datenverarbeitungssystem (28) ausgeführt wird.

13. Computerlesbares Medium, das das Computerprogramm (31) nach Anspruch 12 umfasst.

## Revendications

1. Procédé mis en œuvre par ordinateur de traitement d'une information (1) de mesure pour sélectionner une respective de multiples étiquettes (2, 3) possibles pour de multiples voxels ou pixels (4 à 8) d'un ensemble (9) de données d'image médicale monodimensionnel ou multidimensionnel sur la base d'une information (10) de référence,
- dans lequel l'information (10) de référence comprend un ensemble (11) de données respectif de référence comprenant de multiples valeurs (12) de référence pour chacune des étiquettes (2, 3) possibles,
- dans lequel chaque étiquette (2, 3) est associée à un paramètre (13) de matériau ou à un ensemble (14) de paramètres (13) de matériau d'un tissu, qui est dépeint potentiellement dans l'ensemble (9) de données d'image médicale,
- dans lequel l'étiquette (2, 3) respective pour le voxel ou le pixel (4 à 8) respectif est sélectionnée en utilisant un algorithme (19) discret d'optimisation afin d'optimiser une fonction (15) de coût,
- dans lequel la fonction (15) de coût dépend
• d'une première fonction (16) partielle de coût comprenant une mesure respective de la différence entre un ensemble (18) de données de mesure pour le voxel ou le pixel (4 à 8) correspondant et l'ensemble (11) de données de référence affecté à l'étiquette (2, 3) affectée à ce voxel ou pixel (4 à 8) respectif, et
• une deuxième fonction (17) partielle de coût comprenant une mesure respective de différence pour de multiples paires (21) sélectionnées de voxels ou pixels (4 à 8) différents mutuellement, dans lequel la mesure respective de différence est une mesure de la différence entre les paramètres (13) de matériau ou entre les ensembles (14) de paramètres (13) de matériau associés aux étiquettes (2, 3) sélectionnées pour les voxels ou pixels (4 à 8) de la paire (21) respective,
- dans lequel l'ensemble (18) de données de référence comprend de multiples valeurs (20) de mesure, qui sont ou une valeur (20) unique de mesure qui est sélectionnée ou déterminée à partir de l'information (1) de mesure pour le voxel ou pixel (4 à 8) respectif.

2. Procédé mis en œuvre par ordinateur suivant la revendication 1, **caractérisé en ce que** chacun des voxels ou pixels (4 à 8) de l'ensemble (9) de données d'image médicale fait partie d'au moins l'une des paires (21) sélectionnée de voxels ou de pixels (4 à 8), qui comprend également un autre des voxels ou pixels (4 à 8), qui est voisin du voxel ou pixel (4 à 8).

3. Procédé mis en œuvre par ordinateur suivant la revendication 1 ou 2, dans lequel l'ensemble (9) de données d'image médicale est un ensemble (9) de données d'image médicale au moins bidimensionnel, **caractérisé en ce que** chacun des voxels ou pixels (4 à 8) de l'ensemble (9) de données d'image médicale fait au moins partie d'une ou de deux des paires (21) sélectionnées de voxels ou de pixels (4 à 8), qui comprend également un autre des voxels ou pixels (4 à 8), qui sont voisins au voxel ou pixel (4 à 8) dans la dimension (22, 23) respective pour chacune de la dimension (22, 23) de l'ensemble (9) de données d'image médicale.

4. Procédé mis en œuvre par ordinateur suivant l'une des revendications précédentes, **caractérisé en ce que** l'information (1) de mesure repose sur une séquence (24) de mesure de résonance magnétique.

5. Procédé mis en œuvre par ordinateur suivant la revendication 4, dans lequel le paramètre (13) de matériau ou l'ensemble (14) de paramètres (13) de matériau comprend au moins l'un de : un temps T₁ et/ou un temps T₂ et/ou une densité de spin et/ou une mesure de la quantité d'un tissu spécifique et/ou d'un fluide dans le voxel ou pixel (4 à 8) respectif.

6. Procédé mis en œuvre par ordinateur suivant la revendication 4 ou 5, **caractérisé en ce que** la séquence (24) de mesure de résonance magnétique comprend une séquence d'une séquence (25) partielle, dans lequel l'information (1) de mesure comprend de multiples ensembles (26) de données d'image médicale préliminaires ou dans lequel de multiples ensembles (26) de données d'image médicale préliminaires sont déterminés sur la base de l'information (1) de mesure, dans lequel chacun des ensembles (26) de données d'image médicale préliminaires repose sur une information (27) partielle de mesure acquise pendant une séquence (25) respective partielle, dans lequel chacun des ensembles (26) de données d'image médicale préliminaires donne une respective des multiples valeurs (20) de mesure pour l'ensemble (18) respectif de mesure, dans lequel les séquences (25) partielles diffèrent les unes des autres en ce qui concerne le nombre et/ou l'ordre et/ou les types d'impulsions d'excitation utilisés pendant la séquence (25) respective partielle et/ou concernant la synchronisation des impulsions d'excitation pendant la séquence (25) respective partielle.

7. Procédé mis en œuvre par ordinateur suivant l'une des revendications 4 à 6, **caractérisé en ce que** la séquence (24) de mesure de résonance magnétique est conçue pour donner une information (1) de mesure propre à une évaluation utilisant une empreinte de résonance magnétique ou une différente technique de correspondance par dictionnaire et/ou **en ce que** la sélection de l'étiquette (2, 3) respective pour le voxel ou pixel (4 à 8) respectif repose sur une empreinte de résonance magnétique ou sur une technique différente de correspondance par dictionnaire.

8. Procédé mis en œuvre par ordinateur suivant l'une des revendications 4 à 7, **caractérisé en ce que** l'ensemble (11) de données de référence repose sur des simulations de séquence d'impulsion de résonance magnétique et/ou des équations décrivant des évolutions du signal dans l'imagerie par résonance magnétique.

9. Procédé mis en œuvre par ordinateur suivant l'une des revendications précédentes, **caractérisé en ce que** l'ensemble (11) de données de référence repose sur des mesures de référence et/ou sur une simulation d'une technique (1) d'imagerie, sur laquelle repose l'information (1) de mesure et/ou sur une solution d'équations analytiques décrivant la physique de cette technique d'imagerie.

10. Procédé mis en œuvre par ordinateur suivant l'une des revendications précédentes, **caractérisé en ce que** l'algorithme (19) discret d'optimisation repose sur une approche d'optimisation primale-duale.

11. Système de traitement de données, **caractérisé en ce qu'**il est configuré pour effectuer le procédé mis en œuvre par ordinateur suivant l'une des revendications précédentes.

12. Programme d'ordinateur, **caractérisé en ce qu'**il comprend des instructions pour effectuer le procédé mis en œuvre par ordinateur suivant l'une des revendications 1 à 7, lorsque le programme est exécuté sur un système (28) de traitement de données.

13. Support déchiffrable par ordinateur, comprenant le programme (31) d'ordinateur suivant la revendication 12.
